# EUROPEAN PATENT SPECIFICATION

(11) **EP 0 807 425 B1**
(45) Date of publication and mention of the grant of the patent: **31.07.2002**
(21) Application number: 97303262.6
(22) Date of filing: 13.05.1997
(51) Int. Cl.: A61F 2/06, B29C 53/08

(54) **Making an access graft**
Herstellung eines zugänglichen Transplantats
Fabrication d'une greffe d'accès

(30) Priority: 16.05.1996 GB 9610273
(43) Date of publication of application: 19.11.1997
(73) Proprietor: Nervation Limited, Chester CH1 1HG (GB)
(72) Inventor: Underwood, Christopher John, Denton, Manchester M34 3QD (GB); Charlesworth, David, West Didsbury M20 8LR (GB); Chian, Kerm Sin, 02-33 Singapore 596001 (SG)
(74) Representative: Lawrence, John Gordon

(56) References cited:
- WO-A-93/02637

## Description

This invention relates to making an access graft, for example for use in dialysis patients.

Conventionally, to make a vascular access graft, straight pieces of conventional artificial vascular prostheses, having circular cross-sections, are placed, usually, in the forearm of patients during surgery, and simply bent into a loop *in situ*. However, a major problem is that the artificial vessel may kink at the point where the vessel bends, and, because the vessel is circular, the lumen will totally occlude thus interrupting normal blood flow. This problem may be overcome to some extent by having access grafts with thick walls and small internal diameters which are more kink resistant than those with thinner walls and larger internal diameters. However if the walls of the graft are too thick and lumen too small, the graft will not only have a reduced compliance but also it will be very difficult to repeatedly introduce the large bore needle, used for dialysis purposes.

It has been proposed in WO93/02637 to provide a vascular prosthesis adapted for use as an access graft which has a permanent set, kink-resistant U-bend section. The U-bend is fashioned from a straight, flexible tubular graft which has been made for example by coagulation casting a bio-compatible polymer such as poly (ether) urethane and inserting a bend in the tube when wet, then drying the tube in accordance with the preamble of claim 1.

The achievement of high quality output of access grafts on this basis is problematical, however.

The present invention provides a method by which high quality access grafts can be produced to standard dimensions.

A method for making an access graft comprising a tube of implantable material adapted to be attached in a blood circulatory system and to provide thereby a site for access for cannulation, the tube having a permanent set, kink-resistant U-bend section, the method comprising :
forming a straight tube by a process which yields a set, dryable tube; taking a length of the tube and inserting in it along its length a flexible rod-like support member;
wrapping the tube containing the support member around a core so as to define a bend in the tube, the tube being wet at this stage;
drying the wet tube containing the support member while wrapped around the core whereby permanently to shape the tube into a bent form;
removing the dried tube from the core and removing the support member whereby to form an access graft, U-bend section,
wherein
the core comprises a circular or near-circular rod-like member having a diameter substantially the same as that of the tube and helical fin means in which the tube is located.

The tube may, as before, be one that has been formed by coagulation coating on a former in the fashion described in WO 90/05628.

The tube may be allowed to dry after forming and is then re-wet before the wrapping step. Or the tube can be taken to the wrapping step without ever being allowed to dry.

The support member may be an easy sliding fit in the tube, but should be a reasonably snug fit - its purpose is to prevent kinking - consonant with insertion and removal - primarily insertion. The support member, to ease insertion, is preferably of or is coated with a silicone or like substance that does not stick to the tube. If the tube is wet for the insertion of the support member it is found to have a useful lubricating effect.

The eventual shape of the tube is found to depend on the extent of wrap and for any particular material and configuration it may be necessary to conduct a few trials with different extents of wrap necessary to produce the desired shape.

Typically, however, an appropriately shaped graft will be produced if the tube is wrapped twice around the core.

The wet tube wrapped on the core is constrained against movement (as by hanging weights on the protruding ends of the flexible support member) for drying - taking care not to deform the tube by excessive loading. Drying is preferably effected gently as by leaving overnight in an oven at no more than 40°C.

A method for making an access graft in accordance with the invention will now be described with reference to the accompanying drawings, in which :
- Figure 1: is a drawing showing the insertion of a flexible rod-like support member in a coagulation-cast tubular prosthesis;
- Figure 2: is an elevation of a core on which the tube can be wrapped;
- Figure 3: is an elevation showing the tube in place on the core; and
- Figure 4: is an elevation of the resulting graft.

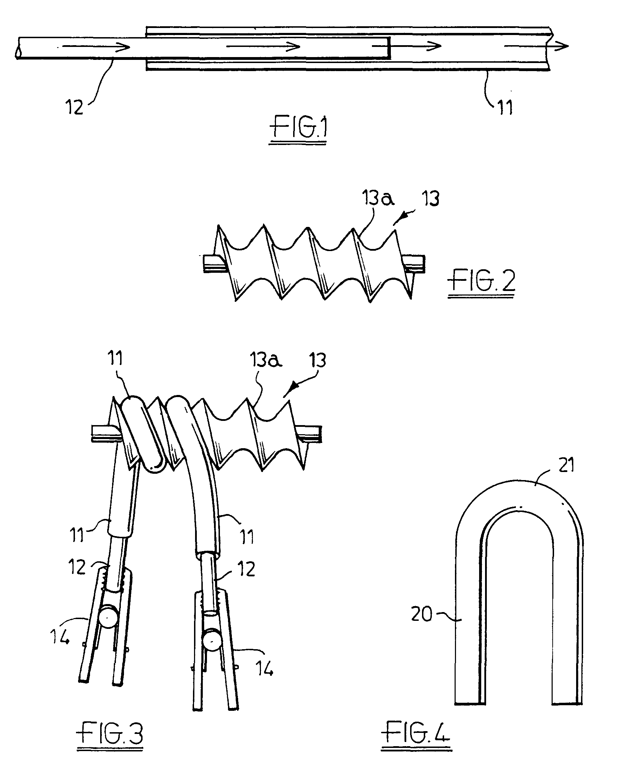

The drawings illustrate a method for making an access graft 20 (Figure 4) comprising a tube of implantable material such as a poly (ether) urethane made as described in WO 90/05628, adapted to be attached in a blood circulatory system and to provide thereby a site 21 for access to cannulation the tube having a permanent set, kink-resistant U-bend section (the site 21), the method comprising :
forming a straight tube 20 by a process (such as that described in WO 90/05628) which yields a wet, dryable tube;
taking a length 11 (Figure 1) of the tube and inserting in it along its length a flexible rod-like support member 12;
wrapping the tube 11 containing the member 12 around a core 13 so as to define a bend in the wet tube - the tube 11 being wet at this stage;
drying the wet tube 11 containing the support member 12 while wrapped around the core 13 whereby permanently to shape the tube 11 into a bent form;
removing the dried tube 11 from the core 13 and removing the support member 12 whereby to form an access graft U-bend section as shown in Figure 4.

The tube 11 may be allowed to dry after being cast according to WO 90/05628 and may be processed dry up to an including its wrapping on the core 13, when it is wetted. However, if the manufacture of access grafts takes place reasonably close in time and space to the initial production of the tube 11 the latter may be taken wet straight from the casting process and never allowed to dry up to and including it being wrapped on the core 13. It is found that the presence of water aids the insertion of the rod-like member 12 and a dry tube 11 might be wetted-out prior to such insertion.

The member 12 should be a reasonably snug fit in the tube 11, but not such as to render threading-on too difficult. Being of or coated with silicone or like material helps insertion and removal.

The core 13 comprises a circular or near circular rod-like member having a diameter substantially the same as that of the tube 11. There is a helical fin 13a in which, as seen in Figure 3, the tube (with its insert member 12) is located while wrapped (twice in this case) around the core 13.

After wrapping on the core 13 (either already wet, or dry, and then wetted-out) the tube 11 is placed in an oven overnight at 40°C (not more, or at least no substantially more) to dry out so as to set the tube 11 so that, on removal from the core 13 and after retrieval of the support member 12, it constitutes a tube 20 with a U-bend section 21.

The wet (and drying) graft wrapped on the core 13 is constrained against movement by hanging weights 14 (or weighted clasps) whose weight is not such as to cause deformation of the graft, and dried overnight at 40°C.

## Claims

1. A method for making an access graft (20) comprising a tube (11) of implantable material adapted to be attached in a blood circulatory system and to provide thereby a site for access for cannulation, the tube having a permanent set, kink-resistant U-bend section (21), the method comprising:
forming a straight tube by a process which yields a set, dryable tube;
taking a length of the tube and inserting in it along its length a flexible rod-like support member (12);
wrapping the tube (11) containing the support member (12) around a core (13) so as to define a bend in the tube (11), the tube being wet at this stage;
drying the wet tube (11) containing the support member (12) while wrapped around the core (13) whereby permanently to shape the tube (11) into a bent form;
removing the dried tube (11) from the core (13) and removing the support
member (12) whereby to form an access graft, U-bend section,
**characterised in that**:
the core (13) comprises a circular or near-circular rod-like member having a diameter substantially the same as that of the tube (11) and helical fin means (13a) in which the tube (11) is located.

2. A method according to claim 1, in which the tube (11) is formed by coagulation casting on a former.

3. A method according to claim 2, in which the tube (11) is allowed to dry after forming and is re-wet before the wrapping step.

4. A method according to claim 2, in which the tube (11) is not allowed to dry between forming and wrapping.

5. A method according to any one of claims 1 to 4, in which the support member (12) is an easy sliding fit in the tube (11).

6. A method according to claim 5, in which the support member (12) is of or is coated with a silicone or like substance that does not stick to the tube (11).

7. A method according to claim 1, in which the tube (11) is wrapped twice around the rod-like member (13).

8. A method according to any one of claims 1 to 7, in which the wrapped, wet tube (11) on the core (13) is constrained against movement (as by weighting protruding ends of the support member) for drying.

9. A method according to any one of claims 1 to 8, in which drying is effected in an oven set a temperature not exceeding 40°C.

## Patentansprüche

1. Verfahren zum Herstellen eines zugänglichen Transplantats (20) mit einem Rohr (11) aus implantierbarem Material, das geeignet ist, in ein Blutkreislaufsystem eingebracht zu werden und eine Stelle für den Zugang einer Kanüle zu liefern, wobei das Rohr einen bleibend verformten, knickfesten, U-förmig gebogenen Abschnitt (21) aufweist, welches Verfahren umfaßt:
Bilden eines geraden Rohres durch einen Prozeß, welcher ein formfestes, trocknungsfähiges Rohr ergibt;
Vorsehen einer bestimmten Länge des Rohres und Einführen eines flexiblem stabartigen Stützgliedes (12) in dieses über dessen Länge;
Wickeln des Rohres (11) enthaltend das Stützglied (12) um einen Kern (13), um eine Biegung in dem Rohr (11) festzulegen, wobei das Rohr in dieser Stufe naß ist;
Trocknen des nassen Rohres (11) enthaltend das Stützglied (12), während es um den Kern (13) gewickelt ist, wodurch das Rohr (11) dauerhaft in eine gebogene Form gebracht ist;
Entfernen des getrockneten Rohres (11) von dem Kern (13) und Entfernen des Stützglieds (12), um hierdurch ein zugängliches Transplantat als U-förmig gebogenen Abschnitt zu bilden,
**dadurch gekennzeichnet, daß**:
der Kern (13) ein kreisförmiges oder nahezu kreisförmiges stabartiges Teil mit einem Durchmesser, der im wesentlichen derselbe wie der des Rohres (11) ist, und eine wendelförmige Steganordnung (13a), in welcher sich das Rohr (11) befindet, aufweist.

2. Verfahren nach Anspruch 1, bei welchem das Rohr (11) durch Koagulationsgießen auf einer Schablone gebildet wird.

3. Verfahren nach Anspruch 2, bei welchem das Rohr (11) nach dem Herstellen trocknen kann und wieder vor dem Wickelschritt genäßt wird.

4. Verfahren nach Anspruch 2, bei welchem das Rohr (11) zwischen Herstellen und Wickeln nicht trocknen kann.

5. Verfahren nach einem der Ansprüche 1 bis 4, bei welchem das Stützglied (12) einen leicht gleitenden Sitz in dem Rohr (11) hat.

6. Verfahren nach Anspruch 5, bei welchem das Stützglied (12) aus Silikon oder einem ähnlichen Stoff besteht oder damit beschichtet ist, welcher nicht an dem Rohr (11) haftet.

7. Verfahren nach Anspruch 1, bei welchem das Rohr (11) zweimal um das stabartige Teil (13) gewunden ist.

8. Verfahren nach einem der Ansprüche 1 bis 7, bei welchem das gewickelte, nasse Rohr (11) zum Trocknen auf dem Kern (13) gegen Bewegung gehalten wird (wie durch Belasten vorstehender Enden des Stützgliedes).

9. Verfahren nach einem der Ansprüche 1 bis 8, bei welchem das Trocknen in einem Ofen, der auf eine 40 Grad C nicht überschreitende Temperatur eingestellt ist, bewirkt wird.

## Revendications

1. Procédé de production d'une greffe d'accès (20) comprenant un tube (11) en matériau implantable conçu pour être fixé dans un système de circulation du sang et pour fournir ainsi un site d'accès pour la canulation, le tube ayant une section courbée en U (21) résistant au vrillage, fixée de manière permanente, le procédé comprenant :
la formation d'un tube rectiligne par un procédé qui produit un tube séchable fixé ;
le prélèvement d'une longueur du tube et l'insertion dans celle-ci, suivant sa longueur, d'un élément de support (12) analogue à une barre flexible ;
l'enroulement du tube (11) contenant l'élément de support (12) autour d'un noyau (13) de manière à définir une courbure dans le tube (11), le tube étant mouillé à ce stade ;
le séchage du tube mouillé (11) contenant l'élément de support (12) tandis qu'il est enroulé autour du noyau (13) pour communiquer de manière permanente au tube (11) une forme courbée ;
le retrait du tube séché (11) du noyau (13) et le retrait de l'élément de support (12) pour former une greffe d'accès à section courbée en U,
**caractérisé en ce que** :
le noyau (13) comprend un élément analogue à une barre circulaire ou sensiblement circulaire ayant un diamètre sensiblement identique à celui du tube (11) et un moyen formant nervure hélicoïdale (13a) dans lequel est situé le tube (11).

2. Procédé selon la revendication 1, où le tube (11) est formé par coulée par coagulation sur un gabarit.

3. Procédé selon la revendication 2, où le tube (11) est mis à sécher après la formation et est remouillé avant l'étape d'enroulement

4. Procédé selon la revendication 2, où le tube (11) n'est pas mis à sécher entre la formation et l'enroulement.

5. Procédé selon l'une quelconque des revendications 1 à 4 où l'élément de support (12) est à ajustement de glissement facile dans le tube (11).

6. Procédé selon la revendication 5 où l'élément de support (12) est constitué par ou est revêtu d'un silicone ou d'une substance analogue qui n'adhère pas au tube (11).

7. Procédé selon la revendication 1 où le tube (11) est enroulé deux fois autour de l'élément analogue à une barre (13).

8. Procédé selon l'une quelconque des revendications 1 à 7 où le tube (11) mouillé enroulé sur le noyau (13) est empêché de se déplacer (par exemple par des extrémités en saillie pesantes de l'élément de support) pour le séchage.

9. Procédé selon l'une quelconque des revendications 1 à 8 où le séchage est réalisé dans une étuve réglée à une température ne dépassant pas 40°C.
